# EUROPEAN PATENT SPECIFICATION

(11) **EP 4 065 216 B1**
(45) Date of publication and mention of the grant of the patent: **09.07.2025**
(21) Application number: 20894751.5
(22) Date of filing: 26.11.2020
(51) Int. Cl.: A61N 1/362, A61N 1/375, A61N 1/05, A61N 1/368, A61B 17/34, A61N 1/365, A61N 1/378, A61N 1/39

(54) **IMPLANTABLE MEDICAL DEVICES FOR MULTI-CHAMBER PACING**
IMPLANTIERBARE MEDIZINPRODUKTE FÜR DIE MEHRKAMMERSTIMULATION
DISPOSITIFS MÉDICAUX IMPLANTABLES POUR UNE STIMULATION MULTICHAMBRE

(30) Priority: 26.11.2019 US 201962940711 P
(43) Date of publication of application: 05.10.2022
(73) Proprietor: Medtronic, Inc., Minneapolis, Minnesota 55432 (US)
(72) Inventor: ASLESON, Andrea J., Minneapolis, Minnesota 55432 (US); DEMMER, Wade M., Minneapolis, Minnesota 55432 (US); GRENZ, Nathan A., Minneapolis, Minnesota 55432 (US); KLEPFER, Ruth N., Minneapolis, Minnesota 55432 (US); MATTSON, Alexander R., Minneapolis, Minnesota 55432 (US); SEIFERT, Kevin, Minneapolis, Minnesota 55432 (US); YANG, Zhongping, Minneapolis, Minnesota 55432 (US)
(74) Representative: Zimmermann & Partner Patentanwälte mbB
(86) International application number: PCT/US2020/062466
(87) International publication number: WO 2021/108744

(56) References cited:
- US-A1- 2004 230 236
- US-A1- 2008 288 008
- US-A1- 2008 288 008
- US-A1- 2010 168 777
- US-A1- 2010 298 841
- US-A1- 2014 172 034
- US-A1- 2015 224 320
- US-A1- 2017 326 372
- US-A1- 2019 192 863
- US-A1- 2019 351 236
- US-A1- 2019 351 236
- US-B2- 10 463 853

## Description

The present technology is generally related to implantable medical devices and, in particular, leadless implantable medical devices and related uses.

US 2019/3512236 A1 relates to systems and methods for threating cardiac arrythmias. US 2004/230236 A1 relates to papillary muscle stimulation.

### SUMMARY

The invention is defined by the independent claim 1.

In one illustrative implantable medical device, the device includes a plurality of electrodes. The plurality of electrodes may include a right atrial electrode positionable within the right atrium to deliver cardiac therapy to or sense electrical activity of the right atrium of the patient's heart and at least one left ventricular electrode positionable proximate the left ventricle of the patient's heart. The device may further include a housing extending from a proximal end region to a distal end region and the right atrial electrode may be leadlessly coupled to the proximal end region. The device may further include a leadlet extending from a proximal region to a distal region, where the proximal region is coupled to the distal end region of the housing and the at least one ventricular electrode is coupled to the distal region of the leadlet. Further, the leadlet may be configured to extend through the coronary sinus ostium and into the coronary sinus or a coronary vein of the patient's heart to position the least one left ventricular electrode proximate the left ventricle of the patient's heart. The device may further include a therapy delivery circuit within the housing and operably coupled to the plurality of electrodes to deliver cardiac therapy to the patient's heart and a sensing circuit within the housing and operably coupled to the plurality of electrodes to sense electrical activity of the patient's heart. The device may further include a controller within the housing and comprising processing circuitry operably coupled to the therapy delivery circuit and the sensing circuit. The controller may be configured to monitor electrical activity using the processing circuitry and one or more of the plurality of electrodes and delivering pacing therapy using the processing circuitry and one or more of the plurality of electrodes.

In one illustrative method, the method may include implanting a right atrial electrode in the right atrial endocardium or in the right atrial myocardium of a patient's heart, the right atrial electrode being leadlessly coupled to a proximal end region of an implantable housing. The method may further include implanting at least one left ventricular electrode through the coronary sinus ostium and into the coronary sinus or a coronary vein of the patient's heart, where the at least one left ventricular electrode being coupled to a distal region of a leadlet and a proximal region of the leadlet being coupled to a distal end region of the implantable housing. Processing circuitry may be located, positioned, or disposed, in the housing and operably coupled to the right atrial electrode and the at least one left ventricular electrode. The method may further include monitoring electrical activity using the processing circuitry and one or more of the right atrial electrode and the at least one left ventricular electrode and delivering pacing therapy using the processing circuitry and at one or more of the right atrial electrode and the at least one left ventricular electrode.

In one illustrative method, the method may include delivering a delivery catheter and a penetration element located, positioned, or disposed, in the delivery catheter to the right ventricular endocardium of the interventricular septal wall of a patient's heart, puncturing the right ventricular endocardium using the penetration element to form an opening through the right ventricular endocardium and into the interventricular septal wall, and retracting the penetration element. The method may further include advancing a distal portion of a guide element through the delivery catheter and the opening and into the interventricular septal wall to extend along the left ventricular endocardial wall and delivering a distal portion of an implantable medical lead over the guide element to the left ventricular myocardium to extend along the left ventricular endocardial wall to position at least one left ventricular electrode on the distal portion in the left ventricular myocardium.

In one illustrative system for delivering an implantable medical lead into the interventricular septal wall and proximate the left ventricular myocardium, the system may include a delivery catheter extending from a proximal end region to a distal end region, where the distal end region positionable adjacent to the right ventricular endocardium of the interventricular septal wall of a patient's heart. The system may further include a penetration element locatable, positionable, or disposable, in the delivery catheter to form an opening through the right ventricular endocardium and into the interventricular septal wall and a guide element extending from a proximal end region to a distal end region. The guide element may be locatable, positionable, or disposable, in the delivery catheter to enter the interventricular septal wall through the opening and to extend along the left ventricular endocardial wall.

The above summary is not intended to describe each embodiment or every implementation of the present disclosure. A more complete understanding will become apparent and appreciated by referring to the following detailed description and claims taken in conjunction with the accompanying drawings. In other words, these and various other features and advantages will be apparent from a reading of the following detailed description.

### BRIEF DESCRIPTION OF THE DRAWINGS

FIG. 1 is a conceptual diagram of one example of a leadless implantable medical device (LIMD) positioned in the right atrium (RA) of a patient's heart including a leadlet positioned in the RA of the patient's heart.
FIGS. 2-3 depicts examples of LIMD including a deployable leadlet.
FIG. 4 is a conceptual diagram of an LIMD positioned in the RA of a patient's heart including a leadlet extending through the coronary sinus (CS) ostium of the patient's heart.
FIGS. 5A-5D are conceptual diagrams showing one example of an implantable medical device (IMD) including an implantable medical lead or leadlet extending through the right ventricular (RV) endocardium into the ventricular septum.
FIGS. 6-7 are conceptual diagrams showing one example of an implantable medical device (IMD) including an implantable medical lead or leadlet extending through the RA endocardium.
FIG. 8 is a conceptual diagram showing one example of a "screw-like" structure that may be used to support and fixate one or more electrodes in myocardial tissue.
FIG. 9 is a block diagram of illustrative circuitry that may be enclosed within a housing of the devices and systems of FIGS. 1-8, for example, to provide the functionality and therapy described herein.
FIG. 10 depicts an illustrative ventricular septum lead and delivery system with components all located with the delivery catheter.
FIG. 11 depicts the ventricular septum lead and delivery system of FIG. 10 with the delivery catheter partially retracted thereby releasing the fixation tines of the outer lead body.
FIG. 12 depicts the ventricular septum lead and delivery system of FIGS. 10-11 with the delivery catheter and the penetrator element both completed removed.
FIG. 13 depicts the ventricular septum lead and delivery system of FIGS. 10-11 with the inner lead body extended from the outer lead body.
FIG. 14 depicts of an illustrative quadripolar lead.

### DETAILED DESCRIPTION

The present disclosure makes reference to a leadless implantable medical device (LIMD). Examples LIMDs having a leadlet that may be used are described in U.S. Patent No. 10,463,853, granted November 5, 2019.

The present disclosure also makes reference to the triangle of Koch region. Examples of uses of the triangle of Koch region are described U.S. Patent Application Publication No. 2019/0290905 published on September 26, 2019.

In general, any suitable type of IMD (or LIMD) including a lead or leadlet may be used. Non-limiting examples of suitable IMDs include an implantable transvenous pacemaker, a transvenous cardiac resynchronization therapy (CRT) device, a transvenous CRT pacemaker (CRT-P), a transvenous CRT defibrillator (CRT-D), an implantable transvenous cardioverter defibrillator (ICD), a subcutaneous ICD (S-ICD), and a subcutaneous medical device.

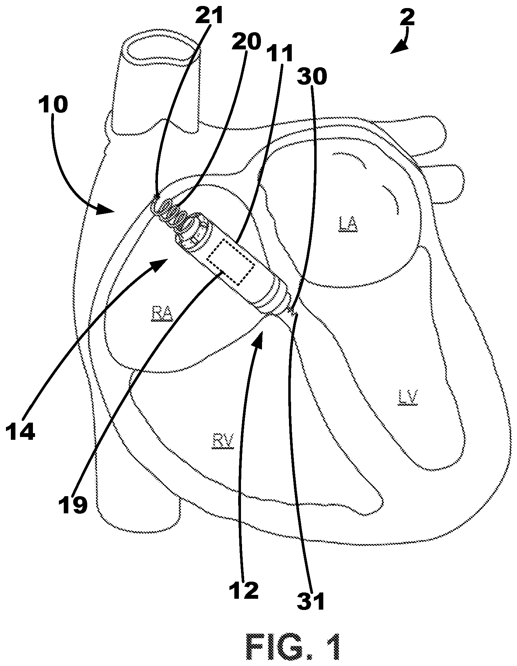

One example of a LIMD 10 positioned in the right atrium (RA) of a patient's heart 2 including a leadlet 20 positioned in the RA of the patient's heart 2 is depicted in FIG. 1. The LIMD 10 may include a housing 11 extending from a distal end region 12 to a proximal end region 14. The leadlet 20 may be physically and operably coupled to the proximal end region 14 of the housing 11 of LIMD 10.

The LIMD 10 may be used to sense electrical activity of the patient's heart or to deliver cardiac therapy to the patient's heart. In general, a first electrode 21 operably and physically coupled to the leadlet 20 may be implanted in the RA endocardium or myocardium. And, a second electrode 31 may be operably and physically coupled to a fixation element 30 extending from the distal end region 12 of the LIMD 10 and may be implanted in the left ventricular (LV) myocardium. More specifically, the second electrode 31 may be coupled to a distal end region 12 of an implantable housing 11 of the LIMD 10, such as disposed on a fixation element 30 (e.g., helical or screw-like structure) extending away from the housing 11 of the LIMD 10 to facilitate fixation. The first electrode 21 may be coupled to a leadlet 20 extending from a proximal end region 14 of the housing 11 of the LIMD 10. The LIMD 10 may be described as a ventricular LIMD implanted in the atrium that paces the ventricle that also includes a leadlet, or "pig tail," that paces the atrium.

The LIMD 10 may be used in any suitable manner. In some embodiments, a method of using the LIMD 10 may include implanting the first, or right atrial, electrode 21 on the right atrial endocardium or in the right atrial myocardium of the patient's heart 2. It may be described that the leadlet 20 extends from a distal end, or first portion, to a proximal end, or second portion. The first, or right atrial, electrode 21 may be coupled to the distal end, or first portion, of the leadlet 20. The proximal end, or second portion, of the leadlet 20 may be coupled to the proximal end region 14 of the housing 11 of the LIMD 10. Processing circuitry 19 may be disposed in the housing 11 and operably coupled to the electrodes 21, 31.

An illustrative method may include implanting a left ventricular electrode through or proximate to the triangle of Koch region of the right atrium, such as in the coronary sinus (CS) ostium, through the right atrial endocardium, and through central fibrous body and into the basal, septal, or basal-septal region in the left ventricular myocardium of the patient's heart. The left ventricular electrode may be leadlessly coupled to a distal portion of the implantable housing. The processing circuitry may be operably coupled to the left ventricular electrode. The method may further include monitoring electrical activity using the processing circuitry and at least one of the right atrial electrode and the left ventricular electrode. Further, the method may include delivering pacing therapy using the processing circuitry and at least one of the right atrial electrode and the left ventricular electrode.

In another embodiment, an LIMD similar to the LIMD 10 of FIG. 1 may be utilized in a different configuration or method. For example, the LIMD may include a right atrial electrode for implantation on the right atrial endocardium or in the right atrial myocardium of the patient's heart, and the right atrial electrode may be leadlessly coupled to a distal end region, or distal portion, of an implantable housing of the LIMD. Further, a left ventricular electrode may be implanted through or proximate to the triangle of Koch region of the right atrium, such as in the CS ostium, through the right atrial endocardium, and through central fibrous body and into the basal, septal, or basal-septal region in the left ventricular myocardium of the patient's heart. The left ventricular electrode may be coupled to a distal end region, or a first portion, of a leadlet. A proximal end region, or a second portion, of the leadlet may be coupled to a proximal end region, or proximal portion, of the implantable housing of the LIMD. The processing circuitry located in the housing may be operably coupled to both of the right and the left ventricular electrode. The LIMD may monitor electrical activity using the processing circuitry and at least one of the right atrial electrode and the left ventricular electrode. Further, the LIMD may deliver pacing therapy using the processing circuitry and at least one of the right atrial electrode and the left ventricular electrode.

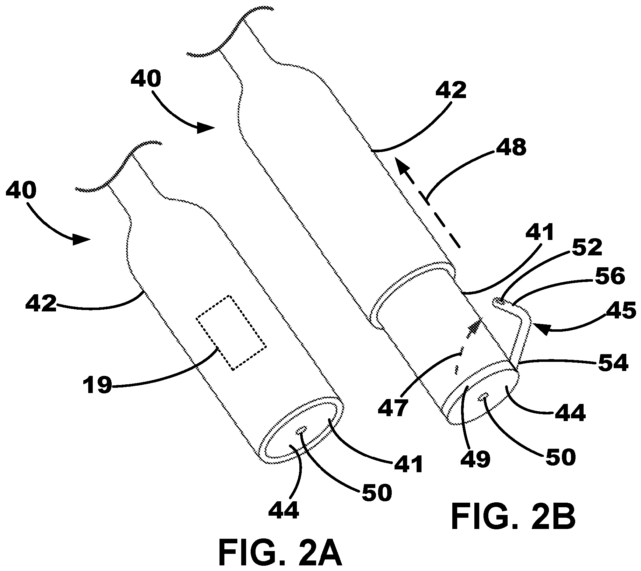

An illustrative LIMD 40 including a deployable leadlet is depicted in FIGS. 2A-2B. The LIMD 40 may include one or more fixation elements (although not shown), a deployable leadlet 45, a first electrode 50, and a second electrode 52 coupled to the leadlet 45. The leadlet 45 extends from a proximal end region 54 to a distal end region 56, and the second electrode is located proximate (or within) the distal end region 56.

The LIMD 40 may include a body portion 41 and a sheath portion 42 moveable with respect to the body portion 41. The sheath portion 42 may define an opening or aperture within which the body portion 41 is located. The LIMD 40 may define a stowed configuration or position as shown in FIG. 2A and a deployed configuration as shown in FIG. 2B. When in the stowed configuration, the body portion 41 may be located substantially completely within the opening of the sheath portion 42 such that, e.g., the deployable leadlet 45 is also in a stowed configuration where the leadlet 45 does not extend beyond or outside of the sheath portion 42. More specifically, when in the stowed configuration, the leadlet 45 may reside within a recess, or groove, 49. The leadlet 45 may include (e.g., be formed of) resilient materials such that, e.g., when the sheath portion 42 is retracted 48 away exposed a distal end region 44 of the body portion 41, the leadlet 45 may move 47 away from (e.g., pivot or partially rotate away from) the body portion 41 in at least partially radial manner to position the distal end region 56, and consequently, the second electrode 52, of the leadlet 45, away from the body portion 41.

Although not shown in FIGS. 2A-2B, one or more fixation elements may be disposed on the distal end region 44 of the body portion 41 of the LIMD 40 and extend distally from the body portion 41. The one or more fixation elements may be electrically active or passive. The first electrode 50 may be positioned on the distal end region 44 of the body portion 41 of the LIMD 40 to engage the endocardial wall of the patient's heart. The second electrode 52 may be coupled to the leadlet 45 extending at least partially in a radial direction away from the body portion 41 in a deployed position. As described herein, the body portion 41 may define, or include, a recess 49 to at least partially receive the leadlet 45 in the stowed position. In other words, it may be described that the body portion 41 may be delivered in a "cup" (e.g., defined by the sheath portion 42) and protracted to deploy the body portion 41 for implantation. In some embodiments, the endocardial wall is the right atrial endocardial wall. Additionally, as can be seen in FIG. 2B, the leadlet 45 may described as defining a shape have two sections or portions. The first section, which is closest to the proximal end region 54, may extend tangentially and in a straight line away from the outside (e.g., circumference) of the body portion 41. The second section, which is closer to the distal end region 56, may then curve or bend away from the tangentially straight line defined by the first section back towards a path somewhat or partially parallel to the outside (e.g., circumference) of the body portion 41.

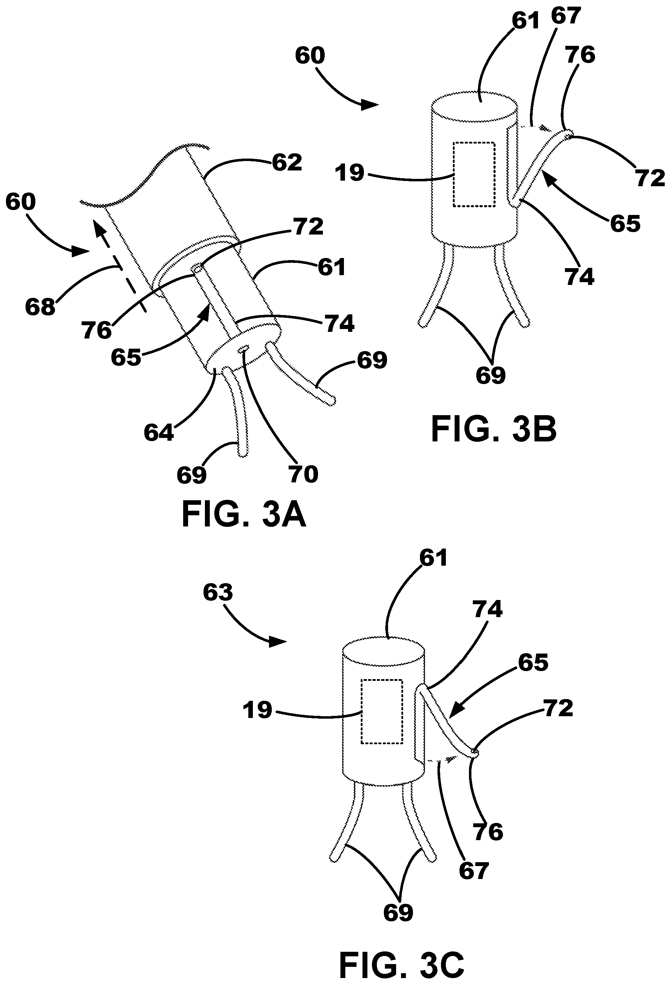

An illustrative LIMD 60 including a deployable leadlet 65 is depicted in FIGS. 3A-3B. The LIMD 60 may include one or more fixation elements 69 (e.g., curved tines), a deployable leadlet 65, a first electrode 70, and a second electrode 72 coupled to the leadlet 65. The leadlet 65 extends from a proximal end region 74 to a distal end region 76, and the second electrode 72 is located proximate (or within) the distal end region 76.

The LIMD 60 may include a body portion 61 and a sheath portion 62 moveable with respect to the body portion 61. The sheath portion 62 may define an opening or aperture within which the body portion 61 is located. The LIMD 60 may define a stowed configuration or position when the body portion 61 is substantially completely within the opening of the sheath portion 62 such that, e.g., the deployable leadlet 65 is also in a stowed configuration or position where the leadlet 65 does not extend beyond or outside of the sheath portion 62. The LIMD 60 may further define a deployed configuration as shown in FIGS. 3A-3B. More specifically, when in the stowed configuration, the leadlet 65 may reside within a recess or groove. The leadlet 65 may include (e.g., be formed of) resilient materials such that, e.g., when the sheath portion 62 is retracted 68 away exposed a distal end region 64 of the body portion 61, the leadlet 65 may move 67 (e.g., pivot or partially rotate) away from the body portion 61 in at least partially radial manner to position the distal end region 76, and consequently, the second electrode 72, of the leadlet 65, away from the body portion 61.

One or more fixation elements 69 may be disposed on the distal end region 64 of the body portion 61 of the LIMD 60 and extend distally from the body portion 61. The one or more fixation elements 69 may be electrically active or passive. The first electrode 70 may be positioned on the distal end region 64 of the body portion 61 of the LIMD 40 to engage the endocardial wall of the patient's heart. The second electrode 72 may be coupled to the leadlet 65 extending at least partially in a radial direction away from the body portion 61 in a deployed position as shown. As described herein, the body portion 61 may define, or include, a recess to at least partially receive the leadlet 65 in the stowed position. In other words, it may be described that the body portion 61 may be delivered in a "cup" (e.g., defined by the sheath portion 62) and protracted to deploy the body portion 61 for implantation. In some embodiments, the endocardial wall is the right atrial endocardial wall.

Another illustrative LIMD 63 including a deployable leadlet 65 is depicted in FIG 3C. The LIMD 63 may be substantially similar to the LIMD 60 except for the movement of the leadlet 65. As shown, the leadlet 65 of LIMD 63 swings, or rotates, away from the body portion 61 in an opposite direction than the leadlet 65 of LIMD 60 to, e.g., provide ease of re-sheathing the body portion 61 into the sheath portion 62. More specifically, the sheath portion 62 may contact and "fold down" the leadlet 65 when the body portion 61 is moved with respect to the sheath portion 62 of the LIMD 63 to position the body portion 61 within the aperture or opening of the sheath portion 62 (i.e., the body portion 61 being retracted into the sheath portion 62). In other words, the LIMD 63 may be provide "easy" retraction of the body portion 61 within the sheath portion 62.

The LIMDs 40, 60, 63 may be used in any suitable manner. In some embodiments, a method of using the LIMDs 40, 60, 63 may include attaching one or more fixation elements 69 thereof to the endocardial wall of the patient's heart. The fixation elements 69 may be coupled to the distal end region, or distal portion, 44, 64 of the body portion 41, 61. Processing circuitry 19 may be disposed in the body portion 41, 61. The method of using the LIMDs 40, 60, 63 may also include implanting the first electrode 50, 70 to engage the endocardial wall of the patient's heart when the fixation element is attached. The first electrode 50, 70 may be leadlessly coupled to the distal end region, or distal portion, 44, 64 of the body portion 41, 61. The processing circuitry 19 may be operably coupled to each of the first and second electrodes 50, 52, 70, 72.

The method of using the LIMDs 40, 60, 63 may further include implanting the second electrode 52, 72 to engage the endocardial wall of the patient's heart when the LIMD 40, 60, 63 is in the deployed configuration placing the leadlet 45, 65 in the deployed position. More specifically, it may be described that the second electrode 52, 72 is coupled to the distal end region, or a first portion, 56, 76 of the leadlet 45, 65 and the proximal end region, or a second portion, 54, 74 of the leadlet 45, 65 may be coupled to the distal end region 44, 64 of the body portion 41, 61. As described herein, the leadlet 45, 65 may extend at least partially in a radial direction away from the body portion 41, 61 in the deployed configuration/position so as to, e.g., engage cardiac tissue with the second electrode 52, 72.

The method of using the LIMDs 40, 60, 63 may further include monitoring electrical activity using the processing circuitry 19 and at least one of the first electrode 50, 70 and the second electrode 52, 72. Further, the method of using the LIMDs 40, 60, 63 may further include delivering pacing therapy using the processing circuitry 19 and at least one of the first electrode 50, 70 and the second electrode 52, 72.

Although no fixation element is depicted on the LIMD 40 in FIGS. 2A-2B and tines 69 are depicted on the LIMD 60 of FIGS. 3A-3C, it is to be understood that any suitable fixation element may be used. In some embodiments, the fixation elements may include one or more helix structures or screw-like structures. In some embodiments, the fixation elements may include one or more adhesives (which may be used in conjunction with other fixation elements such as a helix structure).

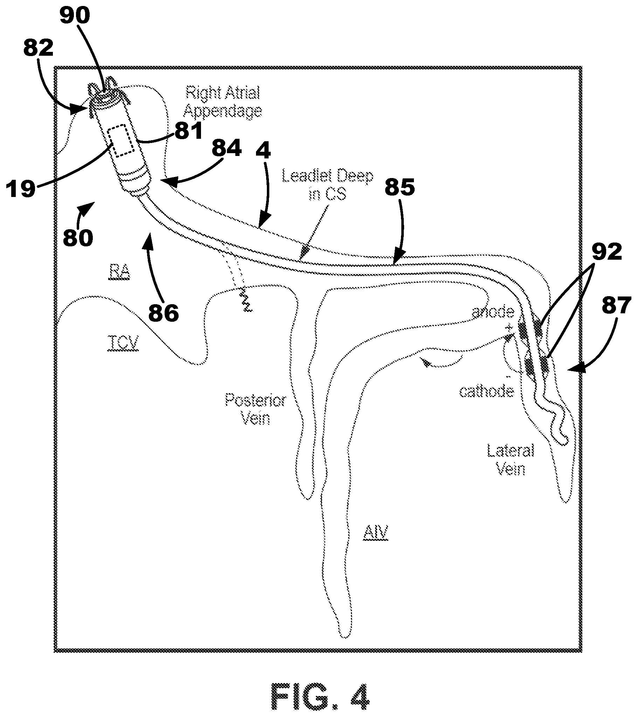

A conceptual diagram of an LIMD 80 positioned in the right atrium of a patient's heart including a leadlet 85 extending through the coronary sinus (CS) ostium of the patient's heart is depicted in FIG. 4. The LIMD 80 may include an implantable housing 81 extending from a proximal end region 82 to a distal end region 84. The LIMD 80 may include a plurality of electrodes. The plurality of electrodes may include one or more right atrial electrodes configured to sense and/or pace right atrial tissue and one or more left ventricular electrodes configured to sense and/or pace left ventricular tissue. For example, as shown, the LIMD 80 includes a right atrial electrode 90 positioned on the proximal end region 82 of the implantable housing 81 of the LIMD 80. The right atrial electrode 90 may be implanted in the right atrial endocardium or in the right atrial myocardium of the patient's heart. In some embodiments, the right atrial electrode 90 may be positioned proximate to (e.g., in contact with, adjacent, partially within, within, etc.) the right atrial appendage.

The LIMD 80 in FIG. 4 also includes a pair of left ventricular electrodes 92 coupled to the leadlet 85 extending distally from the housing 81. The leadlet 85 may be described as extending from a proximal region 86 to a distal region 87. The proximal region 86 is coupled to the distal end region 84 of the housing 81. The electrodes 92 are positioned on or proximate the distal region 87 of the leadlet 85. When implanted, the leadlet 85 may extend through the coronary sinus ostium and into the coronary sinus or a coronary vein of the patient's heart to position the left ventricular electrodes 92 proximate the left ventricle of the patient's heart. Thus, in some embodiments, the left ventricular electrodes 92 may be implanted in the coronary sinus or a coronary vein, and in other embodiments, the left ventricular electrodes may be implanted in the left ventricular myocardium of the patient's heart, for example, using a helical structure or screw-like structure. In the embodiment depicted in FIG. 4, it may be described that a plurality of left ventricular electrodes 92 are coupled to the leadlet 85, for example, including an anode and a cathode. In some embodiments, the plurality of left ventricular electrodes 92 may be used for field steering, for example, to avoid capture of the left atrium (LA) of the patient's heart.

The LIMD 80 may be described as being implanted in the atrium to sense or pace the atrium. The leadlet 85, which may be advanced a relatively long way down the coronary sinus, anterior interventricular vein (AIV) or great cardiac vein (GCV), or lateral vein, to pace the left ventricle or may be advanced a short way into the coronary sinus and then fixated "down" into left ventricular myocardium to pace the left ventricle. In some embodiments, the leadlets described herein may be quadripolar leadlets including four electrodes. Further, any of the leadlets described herein may also use active fixation, which may allow for adjustment of electrode depth, for example, into the left ventricular myocardium.

The LIMD 80 of FIG. 4 may be used in any suitable manner. In some embodiments, a method of using the LIMD 80 may include implanting the right atrial electrode 90 on the right atrial endocardium or in the right atrial myocardium of the patient's heart. The right atrial electrode 90 may be leadlessly coupled to a proximal end region, or portion, 82 of the implantable housing 81. Processing circuitry 19 may be disposed in the housing 81 and may be operably coupled to the right atrial electrode 90.

The method of using the LIMD 80 may also include implanting the left ventricular electrodes 92 through the coronary sinus ostium and into the coronary sinus or a coronary vein of the patient's heart. The left ventricular electrodes 92 may be coupled to the distal region, or first portion, 87 of the leadlet 85. The proximal region, or second portion, 86 of the leadlet 85 may be coupled to the distal end region 84 of the implantable housing 81. The processing circuitry 19 may also be operably coupled to the left ventricular electrodes 92.

The method of using the LIMD 80 may further include monitoring electrical activity using the processing circuitry 19 and at least one of the right atrial electrode 90 and the left ventricular electrodes 92 and may include delivering pacing therapy using the processing circuitry 19 and at least one of the right atrial electrode 90 and the left ventricular electrodes 92. In some embodiments, implanting the left ventricular electrodes 92 may include implanting the left ventricular electrodes 92 into the left ventricular myocardium from the coronary sinus proximal to the posterior vein of the patient's heart. In some embodiments, implanting the left ventricular electrodes 92 may include implanting the left ventricular electrodes 92 into the anterior interventricular vein (AIV) of the patient's heart. In some embodiments, implanting the left ventricular electrodes 92 may include implanting the left ventricular electrodes 92 into the lateral vein of the patient's heart. Further, in some embodiments, delivering pacing therapy may include utilizing field steering to avoid left atrial capture by the left ventricular electrodes 92.

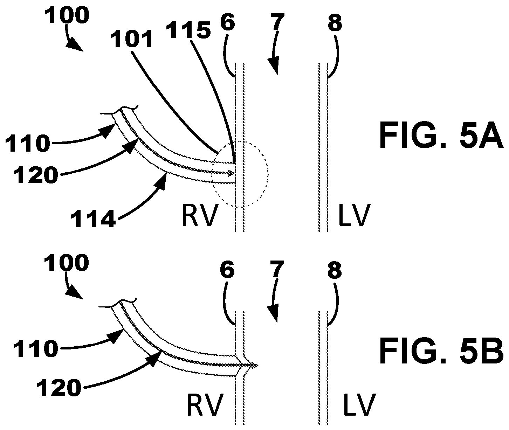

Conceptual diagrams showing one example of implantation of an implantable medical device (IMD) 100 including an implantable medical lead or leadlet 150 extending through the right ventricular (RV) endocardium are depicted in FIGS. 5A-5D. Although a lead is described herein, a leadlet extending from an intracardiac implantable housing of an LIMD may also be used. In other words, the lead 150 could be either a lead extending from an IMD located, or positioned, outside of the heart or a leadlet extending from a LIMD located, or positioned, inside of the heart.

The lead 150 may extend from a proximal portion coupled to the a control portion of the IMD 100 (e.g., a housing, battery, controller, processing circuitry, etc.) to a distal portion 154 that is positioned in or proximate the left ventricular myocardium extending along the left ventricular endocardial wall 8, for example, toward the apex of the patient's heart. The distal portion 154 may be proximate to the left bundle branch (LBB) of the patient's heart. Although any suitable delivery system may be used to deliver the lead 150, a delivery system and method of implantation using such delivery system are shown and described with respect to FIGS. 5A-5D. Generally, the delivery system may be described as puncturing the right ventricular endocardium 6 to allow a lead 150 to be simply pushed into the septal myocardium 7, or interventricular septal wall, and tunneled up or down the septum. The lead 150 may be tunneled into the basal-septal, mid-septal, or even apical septal region with the septal myocardium 7

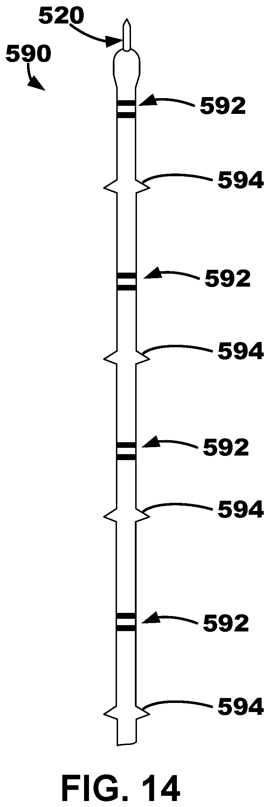

The lead 150 may be a multipolar lead such as, e.g., the quadripolar lead 590 described herein with respect to FIG. 14. More specifically, a distal portion 154 of the lead 150 may have a quadripolar configuration, which may be the same or similar to an ATTAIN STABILITY^{™} or ATTAIN STABILITY QUAD^{™} or ATTAIN PERFORMA^{™} leads available from Medtronic plc of Dublin, Ireland. A side helix or hook usable for fixation that may be disposed on the distal portion, which may be selectively electrically active or passive and may be mechanically active or adjustable. Another exemplary lead that may be employed is described in U.S. Patent No. 9,901,732 to Sommer et al., granted February 27, 2018, in which electrodes are jumpered in a diagonal configuration in order to increase the opportunity to capture cardiac tissue.

A guide element 130, such as a guidewire, a steerable stylet or wire, or a hybrid of stylet guidewire that is a part of the ATTAIN family, may be used to guide 130 the lead 150 into the septum 7. With multiple electrodes and different spacing, one lead 150 may be used to pace the atrium and multiple locations down the septum 7. One example of a guiding element that may be used is described in U.S. Patent No. 7,881,806 to Sommer et al., granted February 1, 2011,

The IMD 100 including the lead 150 may be used in any suitable manner. In some embodiments, a method of delivering and using the IMD 100 may include delivering a delivery catheter 110 and a penetration element 120 disposed in the delivery catheter 110 to the right ventricular endocardium 6 of the interventricular septal wall of a patient's heart as shown in FIG. 5A. The delivery catheter 110 may be configured to provide positioning of the distal end 115 of the delivery catheter to target implantation location 101 such that the distal end 115 is positioned adjacent to (e.g., substantially flush or in contact with) the right ventricular endocardium 6. For example, in one embodiment, the delivery catheter 110 extends from a proximal portion to a distal portion 114, and the distal portion 114 defines a curvature to position the distal end 115 of the catheter 110 substantially flush to the right ventricular endocardium 6.

The method may include puncturing the right ventricular endocardium 6 using the penetration element 120 to form an opening through the right ventricular endocardium 6 and into the interventricular septal wall 7 as shown in FIG. 5B. The penetration element 120 may then be retracted through the delivery catheter 110. Then, a distal portion of a guide element 130 may be advanced through the delivery catheter 110 and the opening and into the interventricular septal wall 7 to extend along the left ventricular endocardial wall 8. The guide element 130 may extend from a proximal region to a distal region 134 and the distal region 134 may be configured to be positioned into the interventricular septal wall 7 to extend along the left ventricular endocardial wall 8. In at least one embodiment, the distal region 134 may define or include a distal curvature portion that curves when exiting the delivery catheter 110 into the interventricular septal wall 7 so as to deliver at least a region of a distal portion 154 of an implantable medical lead 150 substantially parallel to the interventricular septal wall 7 in the left ventricular myocardium.

In addition, a distal portion of the implantable medical lead 150 of the device 100 may be advanced or delivered over the guide element 130 to the left ventricular myocardium to extend along the left ventricular endocardial wall 8 to position one or more left ventricular electrodes 162 on the distal portion in the left ventricular myocardium. In one or more embodiments, the guide element 130 does not extend beyond the tip of the lead 150, and instead, may remain a selected distance away from the tip (e.g., about 1 centimeter) to allow tip to flex during blunt dissection/tunneling of the lead150. Further, Examples of the guide element 130 may include a steerable stylet, shaped stylets, etc. In some embodiments, one or more left ventricular electrodes 162 may be positioned proximate to the left bundle branch (LBB) of the conduction system of the patient's heart.

In some embodiments, a right ventricular electrode 160 on the lead 150 may be positioned/implanted proximate to the right ventricular endocardium 6. The right ventricular electrode 160 may be positioned proximal to the left ventricular electrode on the distal portion 154 of the implantable medical lead 150.

Further, the method of using IMD 100 may include monitoring electrical activity of at least one of the right ventricular electrode 160 and one or more of the left ventricular electrodes 162, and delivering pacing therapy using at least one of the right ventricular electrode 160 and the left ventricular electrodes 162. In some embodiments, delivering pacing therapy may include delivering pacing pulses to the left ventricular electrodes 162 configured to pace the left bundle branch of the conduction system of the patient's heart. In some embodiments, the implantable medical lead 150 may include a plurality of left ventricular electrodes, a plurality of right ventricular electrodes, or both.

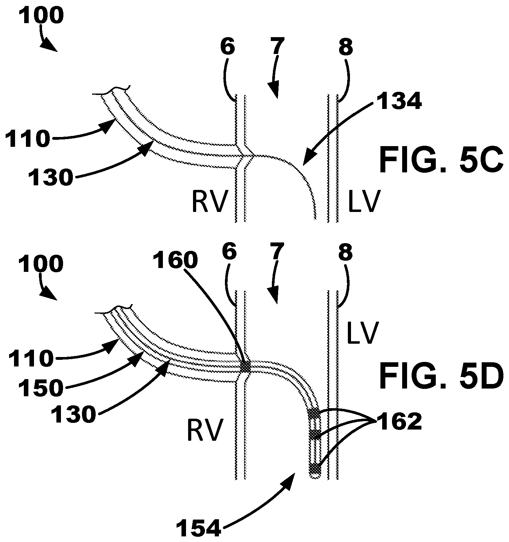

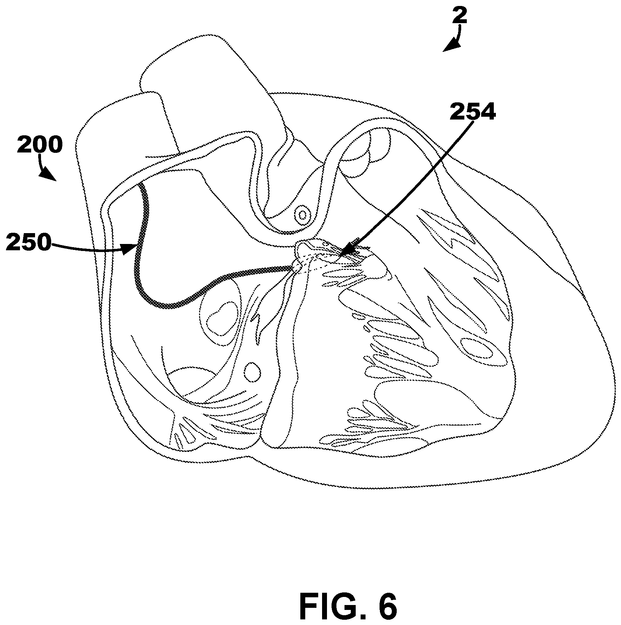

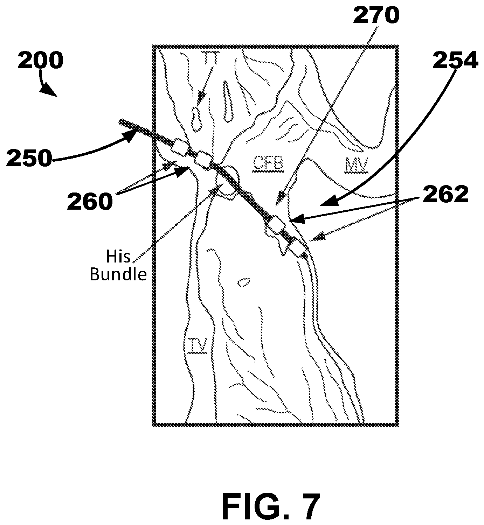

FIGS. 6-7 are conceptual diagrams showing one example of an implantable medical device (IMD) 200 including an implantable medical lead or leadlet 250 extending through the right atrial endocardium. Although a lead 250 is described herein, a leadlet extending from an intracardiac implantable housing may also be used. A distal portion 254 of the lead 250 may be positioned in the left ventricular myocardium extending along the left ventricular endocardial wall, for example, toward the apex of the patient's heart. The distal portion 254 may be proximate to the left bundle branch (LBB) of the patient's heart 2. Any suitable delivery system and method may be used to deliver the lead 250. In at least one embodiment, the delivery systems described herein with respect to FIG. 5 and FIGS. 10-14 may be utilized. For example, a delivery system may be configured to puncture the right atrial endocardium and central fibrous body (CFB) to allow the lead 250 to be simply pushed into the septal myocardium, or interventricular septal wall, and tunneled up or down the septum as shown by the trajectory 270 in FIG. 7. The lead 250 may be tunneled into the basal-septal, mid-septal, or even apical septal region.

A multipolar lead may be used such as, e.g., the quadripolar lead 590 described herein with respect to FIG. 14. The distal portion 254 of the lead 250 may have a quadripolar configuration, which may be the same or similar to an ATTAIN STABILITY^{™} or ATTAIN STABILITY QUAD^{™} or ATTAIN PERFORMA^{™} leads available from Medtronic plc of Dublin, Ireland. A side helix or hook usable for fixation that may be disposed on the distal portion, which may be selectively electrically active or passive and may be mechanically active or adjustable. Another exemplary lead that may be employed is described in U.S. Patent No. 9,901,732 to Sommer et al., granted February 27, 2018, in which electrodes are jumpered in a diagonal configuration in order to increase the opportunity to capture cardiac tissue.

The guiding element may be a guidewire, a steerable stylet or wire, or a hybrid of stylet guidewire that is a part of the ATTAIN family, may be used to guide the lead into the septum. With multiple electrodes and different spacing, one lead may be used to pace the atrium and multiple locations down the septum. One example of a guiding element that may be used is described in U.S. Patent No. 7,881,806 to Sommer et al., granted February 1, 2011

The IMD 200 may be used in any suitable manner. In some embodiments, a method of implanting and using the IMD 200 may include delivering the delivery catheter and a penetration element disposed in the delivery catheter to the right atrial endocardium in or proximate to the triangle of Koch region of a patient's heart, such as in the coronary sinus ostium. The method of implanting and using the IMD 200 may also include puncturing the right atrial endocardium and the central fibrous body using the penetration element to form an opening through the right ventricular endocardium and the central fibrous body and into the interventricular septal wall. Then, the penetration element may be retracted. Then, a distal portion of a guide element may be advanced through the delivery catheter and the opening and into the interventricular septal wall to extend along the left ventricular endocardial wall.

In addition, the method of implanting and using the IMD 200 may include delivering a distal portion 254 of an implantable medical lead 250 over the guide element to the left ventricular myocardium to extend along the left ventricular endocardial wall to position one or more left ventricular electrodes 262 on the distal portion 254 in the left ventricular myocardium. In some embodiments, the left ventricular electrodes 262 may be positioned proximate to the left bundle branch of the conduction system of the patient's heart. In some embodiments, the method of implanting and using the IMD 200 may also include implanting one or more right atrial electrodes 260 proximate to the right atrial endocardium. The right atrial electrodes 260 may be positioned proximally along the lead 250 to the left ventricular electrodes 262 on the distal portion 254 of the implantable medical lead 250.

In some embodiments, the method of using the IMD 100 may further include monitoring electrical activity of at least one of the right atrial electrodes 260 and the left ventricular electrodes 262, and delivering pacing therapy using at least one of the right atrial electrodes 260 and the left ventricular electrodes 262. In some embodiments, delivering pacing therapy may include delivering pacing pulses to the left ventricular electrodes 262 configured to pace the left bundle branch of the conduction system of the patient's heart. Further, In some embodiments, the implantable medical lead may include a plurality of left ventricular electrodes, a plurality of right atrial electrodes, or both.

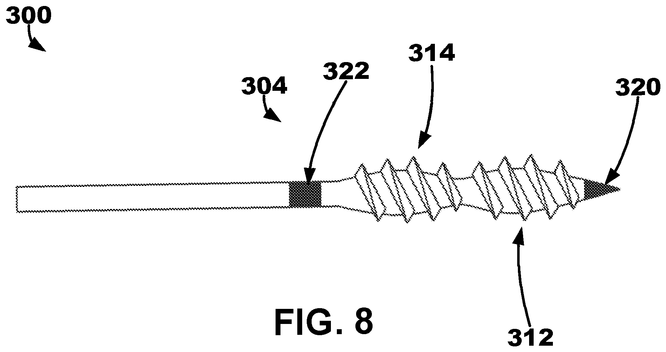

A conceptual diagram showing one example of a "screw-like" structure that may be used to support and fixate one or more electrodes in myocardial tissue is depicted in FIG. 8. In some embodiments, IMD or LIMD may include an elongate member 300 extending between a proximal portion and a distal portion 304. A first electrode 320 may be disposed on the distal portion 304 of the elongate member. A second electrode 322 may be disposed proximal to the first electrode 320 on the distal portion 304 of the elongate member 300. A first threaded bulb region 312 may be disposed between the first and second electrodes 320, 322 on the distal portion 304 of the elongate member 300. A second threaded bulb region 314 disposed between the first threaded bulb region 312 and the second electrode 322 on the distal portion 304 of the elongate member 300. In some embodiments, the threaded bulb regions 312, 314 may share a similar threaded pattern or spacing.

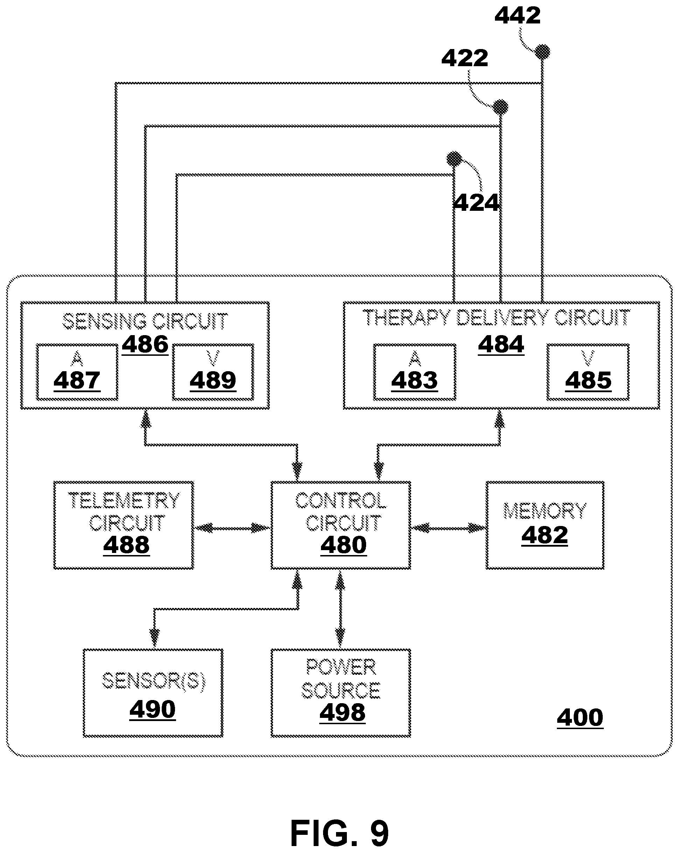

FIG. 9 is a block diagram of circuitry that may be enclosed within the housings of an illustrative IMD 400 to provide the functions of cardiac therapy described herein with respect to FIGS. 1-8. In other words, the IMD 400 may be used with any one or more of the embodiments described with respect to FIGS. 1-8. The electronic circuitry of the device 400 may include software, firmware, and hardware that cooperatively monitor atrial and ventricular electrical cardiac signals, determine when a cardiac therapy is necessary, and/or deliver electrical pulses to the patient's heart according to programmed therapy mode and pulse control parameters. The electronic circuitry may include a control circuit 480 (e.g., including processing circuitry), a memory 482, a therapy delivery circuit 484, a sensing circuit 486, and/or a telemetry circuit 488. In some examples, the device 400 includes one or more sensors 490 for producing a signal that is correlated to a physiological function, state, or condition of the patient, such as a patient activity sensor, for use in determining a need for pacing therapy and/or controlling a pacing rate.

The power source 498 may provide power to the circuitry of the device 400 including each of the components 480, 482, 484, 486, 488, and 490, as needed. The power source 498 may include one or more energy storage devices, such as one or more rechargeable or non-rechargeable batteries. The connections between the power source 498 and each of the components 480, 482, 484, 486, 488, and 490 are to be understood from the general block diagram illustrated but are not shown for the sake of clarity. For example, the power source 498 may be coupled to one or more charging circuits included in the therapy delivery circuit 484 for providing the power needed to charge holding capacitors included in the therapy delivery circuit 484 that are discharged at appropriate times under the control of the control circuit 480 for delivering pacing pulses, e.g., according to a dual chamber pacing mode such as DDI(R). The power source 498 may also be coupled to components of the sensing circuit 486, such as sense amplifiers, analog-to-digital converters, switching circuitry, etc., sensors 490, the telemetry circuit 488, and the memory 482 to provide power to the various circuits.

Any suitable technique may be used to recharge a rechargeable power source 498. In some embodiments, the device 400 may include an antenna, inductive coils, or other inductive coupling structures configured to couple to another device, such as an external charger or programmer, to receive power in situ. Various examples of charging a leadless implantable medical device are described in U.S. Patent Pub. No. 2018/0212451 (Schmidt et al.), filed January 26, 2017, entitled "Recharge of Implanted Medical Devices,". The device 400 may also be configured to use various techniques to extend the life of the power source 498, such as a low-power mode.

Various examples of power sources and techniques related to power sources may be used, such as those found in, for example, U.S. Patent No. 8,383,269 (Scott et al.), granted February 26, 2013, U.S. Patent No. 8,105,714 (Schmidt et al.), granted January 31, 2012, and U.S. Patent No. 7,635,541 (Scott et al.), granted December 22, 2009.

The functional blocks shown represent functionality included in the device 400 and may include any discrete and/or integrated electronic circuit components that implement analog, and/or digital circuits capable of producing the functions attributed to the device 400 herein. The various components may include processing circuitry, such as an application specific integrated circuit (ASIC), an electronic circuit, a processor (shared, dedicated, or group), and memory that execute one or more software or firmware programs, a combinational logic circuit, state machine, or other suitable components or combinations of components that provide the described functionality. The particular form of software, hardware, and/or firmware employed to implement the functionality disclosed herein will be determined primarily by the particular system architecture employed in the medical device and by the particular detection and therapy delivery methodologies employed by the medical device. Providing software, hardware, and/or firmware to accomplish the described functionality in the context of any modern cardiac medical device system, given the disclosure herein, is within the abilities of one of skill in the art.

The memory 482 may include any volatile, non-volatile, magnetic, or electrical non-transitory computer readable storage media, such as random-access memory (RAM), read-only memory (ROM), non-volatile RAM (NVRAM), electrically-erasable programmable ROM (EEPROM), flash memory, or any other memory device. Furthermore, the memory 482 may include a non-transitory computer readable media storing instructions that, when executed by one or more processing circuits, cause the control circuit 480 and/or other processing circuitry to perform a single, dual, or triple chamber pacing (e.g., single or multiple chamber pacing) function or other sensing and therapy delivery functions attributed to the device 400. The non-transitory computer-readable media storing the instructions may include any of the media listed above.

The control circuit 480 may communicate, e.g., via a data bus, with the therapy delivery circuit 484 and the sensing circuit 486 for sensing cardiac electrical signals and controlling delivery of cardiac electrical stimulation therapies in response to sensed cardiac events, e.g., P-waves and R-waves, or the absence thereof. The electrodes 422, 424, 442 (e.g., left ventricular electrodes, right ventricular electrodes, right atrial electrodes, housing electrodes, etc.) may be electrically coupled to the therapy delivery circuit 484 for delivering electrical stimulation pulses to the patient's heart and to the sensing circuit 486 and for sensing cardiac electrical signals.

The sensing circuit 486 may include an atrial (A) sensing channel 487 and a ventricular (V) sensing channel 489. For example, the electrodes 422, 424 may be coupled to the atrial sensing channel 487 for sensing atrial signals, e.g., P-waves attendant to the depolarization of the atrial myocardium. Further, in some examples, the sensing circuit 486 may include switching circuitry for selectively coupling one or more of the available electrodes to cardiac event detection circuitry included in the atrial sensing channel 487. Switching circuitry may include a switch array, switch matrix, multiplexer, or any other type of switching device suitable to selectively couple components of the sensing circuit 486 to selected electrodes. Further, for example, electrodes 424, 442 may be coupled to the ventricular sensing channel 489 for sensing ventricular signals, e.g., R-waves attendant to the depolarization of the ventricular myocardium.

Each of the atrial sensing channel 487 and the ventricular sensing channel 489 may include cardiac event detection circuitry for detecting P-waves and R-waves, respectively, from the cardiac electrical signals received by the respective sensing channels. The cardiac event detection circuitry included in each of the channels 487 and 489 may be configured to amplify, filter, digitize, and rectify the cardiac electrical signal received from the selected electrodes to improve the signal quality for detecting cardiac electrical events. The cardiac event detection circuitry within each channel 487 and 489 may include one or more sense amplifiers, filters, rectifiers, threshold detectors, comparators, analog-to-digital converters (ADCs), timers, or other analog or digital components. A cardiac event sensing threshold, e.g., a P-wave sensing threshold and an R-wave sensing threshold, may be automatically adjusted by each respective sensing channel 487 and 489 under the control of the control circuit 480, e.g., based on timing intervals and sensing threshold values determined by the control circuit 480, stored in the memory 482, and/or controlled by hardware, firmware, and/or software of the control circuit 480 and/or the sensing circuit 486.

Upon detecting a cardiac electrical event based on a sensing threshold crossing, the sensing circuit 486 may produce a sensed event signal that is passed to the control circuit 480. For example, the atrial sensing channel 487 may produce a P-wave sensed event signal in response to a P-wave sensing threshold crossing. The ventricular sensing channel 489 may produce an R-wave sensed event signal in response to an R-wave sensing threshold crossing. The sensed event signals may be used by the control circuit 480 for setting pacing escape interval timers that control the basic time intervals used for scheduling cardiac pacing pulses. A sensed event signal may trigger or inhibit a pacing pulse depending on the particular programmed pacing mode. For example, a P-wave sensed event signal received from the atrial sensing channel 487 may cause the control circuit 480 to inhibit a scheduled atrial pacing pulse and schedule a ventricular pacing pulse at a programmed atrioventricular (AV) pacing interval. If an R-wave is sensed before the AV pacing interval expires, the ventricular pacing pulse may be inhibited. If the AV pacing interval expires before the control circuit 480 receives an R-wave sensed event signal from the ventricular sensing channel 489, the control circuit 480 may use the therapy delivery circuit 484 to deliver the scheduled ventricular pacing pulse synchronized to the sensed P-wave.

In some examples, the device 400 may be configured to deliver a variety of pacing therapies including bradycardia pacing, cardiac resynchronization therapy, post-shock pacing, and/or tachycardia-related therapy, such as ATP, among others. For example, the device 40 may be configured to detect non-sinus tachycardia and deliver ATP. The control circuit 480 may determine cardiac event time intervals, e.g., P-P intervals between consecutive P-wave sensed event signals received from the atrial sensing channel 487, RR intervals between consecutive R-wave sensed event signals received from the ventricular sensing channel 489, and P-R and/or R-P intervals received between P-wave sensed event signals and R-wave sensed event signals. These intervals may be compared to tachycardia detection intervals for detecting non-sinus tachycardia. Tachycardia may be detected in a given heart chamber based on a threshold number of tachycardia detection intervals being detected.

The therapy delivery circuit 484 may include atrial pacing circuit 483 and ventricular pacing circuit 485. Each pacing circuit 483 and 485 may include charging circuitry, one or more charge storage devices such as one or more low voltage holding capacitors, an output capacitor, and/or switching circuitry that controls when the holding capacitor(s) are charged and discharged across the output capacitor to deliver a pacing pulse to the pacing electrode vector coupled to respective pacing circuits 483 or 485. The electrodes 424, 442 may be coupled to the ventricular pacing circuit 485 as a bipolar cathode and anode pair for delivering ventricular pacing pulses, e.g., upon expiration of an AV or VV pacing interval set by the control circuit 480 for providing atrial-synchronized ventricular pacing and a basic lower ventricular pacing rate.

The atrial pacing circuit 483 may be coupled to, for example, the electrodes 422, 424 to deliver atrial pacing pulses. The control circuit 480 may set atrial pacing intervals according to a programmed lower pacing rate or a temporary lower rate set according to a rate-responsive sensor indicated pacing rate. Atrial pacing circuit may be controlled to deliver an atrial pacing pulse if the atrial pacing interval expires before a P-wave sensed event signal is received from the atrial sensing channel 487. The control circuit 480 starts an AV pacing interval in response to a delivered atrial pacing pulse to provide synchronized multiple chamber pacing (e.g., dual or triple chamber pacing).

Charging of a holding capacitor of the atrial or ventricular pacing circuit 483 or 485 to a programmed pacing voltage amplitude and discharging of the capacitor for a programmed pacing pulse width may be performed by the therapy delivery circuit 484 according to control signals received from the control circuit 480. For example, a pace timing circuit included in the control circuit 480 may include programmable digital counters set by a microprocessor of the control circuit 480 for controlling the basic pacing time intervals associated with various single chamber or multiple chamber pacing (e.g., dual or triple chamber pacing) modes or anti-tachycardia pacing sequences. The microprocessor of the control circuit 480 may also set the amplitude, pulse width, polarity, or other characteristics of the cardiac pacing pulses, which may be based on programmed values stored in the memory 482.

The device 400 may include other sensors 490 for sensing signals from the patient for use in determining a need for and/or controlling electrical stimulation therapies delivered by the therapy delivery circuit 484. In some examples, a sensor indicative of a need for increased cardiac output may include a patient activity sensor, such as an accelerometer. An increase in the metabolic demand of the patient due to increased activity as indicated by the patient activity sensor may be determined by the control circuit 480 for use in determining a sensor-indicated pacing rate.

Control parameters utilized by the control circuit 480 for sensing cardiac events and controlling pacing therapy delivery may be programmed into the memory 482 via the telemetry circuit 488, which may also be described as a communication interface. The telemetry circuit 488 includes a transceiver and antenna for communicating with an external device such as a programmer or home monitor, using radio frequency communication or other communication protocols. The control circuit 480 may use the telemetry circuit 488 to receive downlink telemetry from and send uplink telemetry to the external device. In some cases, the telemetry circuit 488 may be used to transmit and receive communication signals to/from another medical device implanted in the patient.

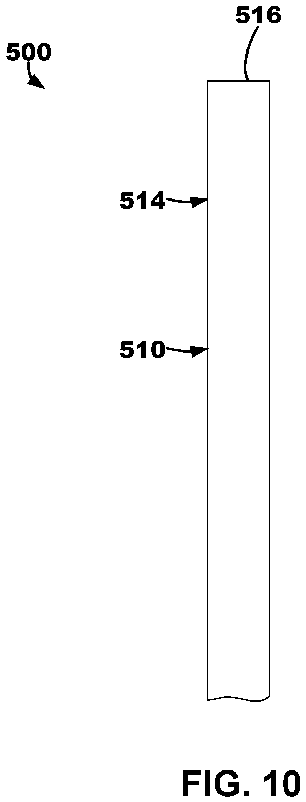

An illustrative ventricular septum lead and delivery system 500 is depicted in FIGS. 10-14 that may be used in a similar manner as the illustrative method and system described herein with respect to FIG. 4 The lead and delivery system 500 may include a delivery catheter 510 that extends from a proximal end region (not depicted) to a distal end region 514. The delivery catheter 510 may define an opening, or aperture, extending from a proximal end to a distal end 516. The other components of the lead and delivery system 500 may be located, or positioned, within the opening of the delivery catheter 510 for delivery to a target location such as right ventricular endocardium, the ventricular septum, etc.

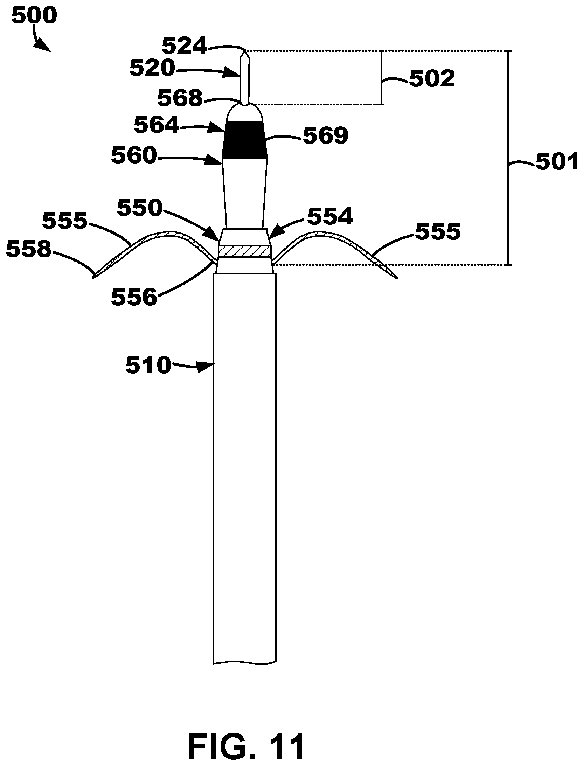

The delivery catheter 510 may be manipulable by a clinician to locate, or position, the distal end 516 proximate (e.g., adjacent, in close contact with, substantially flush to, etc.) a target location such as, e.g., the right ventricular endocardium of the interventricular septal wall of a patient's heart, the right atrial endocardium and central fibrous body (CFB), etc. When the delivery catheter 510 is positioned proximate the target location, the delivery catheter 510 may be retracted thereby exposing an penetration element 520 located within an inner lead body 560, which is located in an outer lead body 550, as shown in FIG. 11. In other words, the delivery catheter 510 may be moved relative to the remaining components of the system 500. In at least one embodiment, the remaining components of the system 500 may be held, or kept, secure and stationary while the delivery catheter 510 is pulled proximally away from the target location.

The outer lead body 550 extends from a proximal end region to a distal end region 554 and includes, among other things, fixation elements 555 located proximate the distal end region 554. The fixation elements 555 may be resilient such that, e.g., they may be "folded up" within the delivery catheter 510 and may move, or "spring," to a deployed state as shown in FIG. 11. In other words, the fixation elements 555 may be configured in a stowed configuration and a deployed configuration. The fixation elements 555 may be predisposed in the deployed configuration and configured to move back to the deployed configuration without application of an outside force. Thus, the delivery catheter 510 may provide an outside force by contacting the fixation elements 555 to hold (e.g., "fold down," restrict, etc.) the fixation elements 555 in the stowed configuration when the distal end region 554 is located within the opening of the delivery catheter 510. More specifically, each of the fixation elements extends from a proximal end 556 attached to the distal end region 554 to a distal end 558, and when the stowed configuration, the distal end 558 may extend and point to the distal end 516 of the delivery catheter 510. When the distal end 516 of the delivery catheter 510 is adjacent tissue at a target location and the delivery catheter 510 is moved to release the fixation elements 555 of the outer lead body 550, the fixation elements 555 may pierce the tissue at the target location and "pull" the outer lead body 550 (and inner lead body 560 and penetration element 520) into the tissue. Such fixation element configuration is described further in U.S. Pat. App. Pub. No. 2019/0076646 A1 entitled "Securing an Implantable Medical Device In Position while Reducing Perforations" published on March 14, 2019, Although two fixation elements 555 are depicted in FIGS. 11-13, it is be understood that one fixation element or more than two fixation elements may be used with the illustrative systems and methods described herein.

A distance 501 may be defined between where the proximal end 556 of the fixation element 555 is coupled to (e.g., extends from) the outer lead body 550 and a distal penetration end 524 of the penetration element 520. The distance 501 may be between about 2 millimeters (mm) and about 15 mm. In one embodiment, the distance 501 may be 4 mm. In one or more embodiments, the distance 501 may be greater than or equal to 2 mm, greater than or equal to 5 mm, greater than or equal to 8 mm, etc. and/or less than or equal to 4 mm, less than or equal to 7 mm, less than or equal to 10 mm, less than or equal to 12 mm etc.

In some embodiments, the distance 501 may be referred to as an initial penetration distance because, for example, the force applied to the inner lead body 560 and penetration element 520 by the fixation elements 555 being released in the deployed positioned and "grabbing" tissue may be sufficient to drive the inner lead body 560 and penetration element 520 into the target location up to the distal end region 554 of the outer lead body 550. In other words, the fixation elements 555 may "drive" the inner lead body 560 and penetration element 520 into the tissue across the distance 501.

Further, a relationship between the distance 501 and the length and shape of the fixation element 555 (i.e., from the proximal end 556 to the distal end 558) may be defined to provide effective penetration to a particular target location. For example, the length of the fixation elements 555 may be less than the distance 501 by about 20% or mm to, e.g., allow the penetration element 530 and the inner lead body 560 (e.g., at least the distal end 568) to contact and/or penetrate the endocardium prior the fixation elements 555 contacting the endocardium.

The outer lead body 550 may include one or more electrodes within the distal end region 554. In at least one embodiment, the fixation elements 555 may function as electrodes to, e.g., sense and or pace right ventricular tissue when the fixation elements 555 are located in the right ventricular endocardium.

The outer lead body 550 defines an opening extending from the proximal end region to the distal end region 554, and as shown in FIGS. 11-13, the inner lead body 560 may be located therein. The inner lead body 560 may also extend from a proximal end region to a distal end region 564 and may define an opening extending from the proximal end region to the distal region 564, within which the penetration element 520, a guide wire, or stylet may be inserted (e.g., located therethrough). As shown in FIG. 11, a penetration element 520 extends from a proximal end to the distal penetration end 524 and is located in the opening of the inner lead body 560 extending distally from a distal end 568 of the inner lead body 560.

The system 500 may be configured to restrict, or limit, the distance 502 that the penetration element 520 may extend beyond the distal end 568 of the inner lead body 560. The distance 502 may be defined between the distal end 568 of the inner lead body 560 and the distal penetration end 524 of the penetration element 520 (e.g., a sharpened point configured for penetration for the right ventricular endocardium). The distance 502 may be between about 1 millimeter (mm) and about 5 mm. In one embodiment, the distance 502 may be 2 mm. In one or more embodiments, the distance 502 may be greater than or equal to 1 mm, greater than or equal to 1.5 mm, greater than or equal to 2.5 mm, etc. and/or less than or equal to 5 mm, less than or equal to 4 mm, less than or equal to 3.5 mm, less than or equal to 3 mm etc.

The inner lead body 560 may further include one or more electrodes 569 that may be configured to be positioned within the intraventricular septum to sense cardiac electrical activity and deliver pacing therapy (e.g., cardiac conduction system pacing therapy, left bundle branch pacing therapy, etc.). Only one of the electrodes 569 is exposed on FIG. 11.

Once the outer lead body 550 is fixated to the target location such as, e.g., the right ventricular endocardial wall, the inner lead body 560 may be moved relative to the outer lead body 550 to implant the distal end region 564 of the inner lead body 560 within the ventricular septum. More specifically, the outer lead body 550 may remain relatively stationary as being fixated, or anchored, to tissue at the target location. The penetration element 520 will have been used to penetrate the target location (e.g., the right ventricular endocardium), and thus, the inner lead body 560 may then follow the opening made by the penetration element 520 (e.g., tunnel into the opening). First, the penetration element may be removed (e.g., retracted proximally) from the inner lead body 560. In some embodiments, the inner lead body 560 may then be moved relative the outer lead body 550 to position to the distal end region 564 in the desired location for sensing and/or pacing (e.g., with the intraventricular septum proximate the left ventricular endocardium with puncturing or penetrating the left ventricular endocardium). In other embodiments, a guide wire or stylet may be inserted through the opening of the inner lead body 560, which may then be used to position the distal end region 564 of the inner lead body 560 in the desired location. Additionally, the distal end region 564 and distal end 568 of the inner lead body 560 may define a curvature or taper configured to provide tunneling functionality into tissue and to effectively expand the opening made by the penetration element 520.

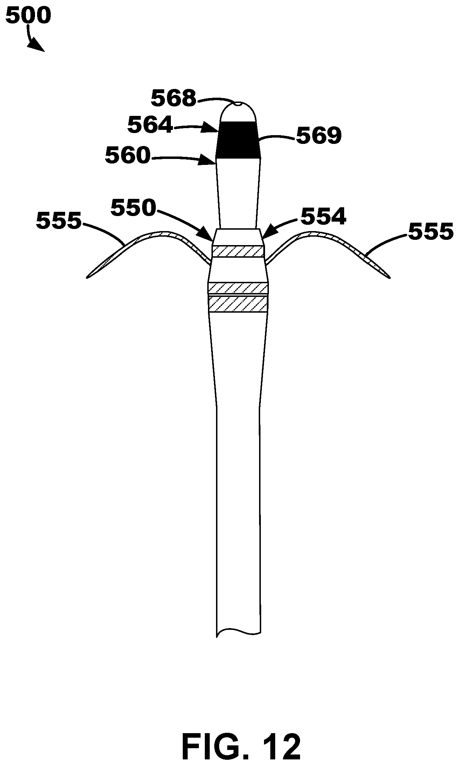

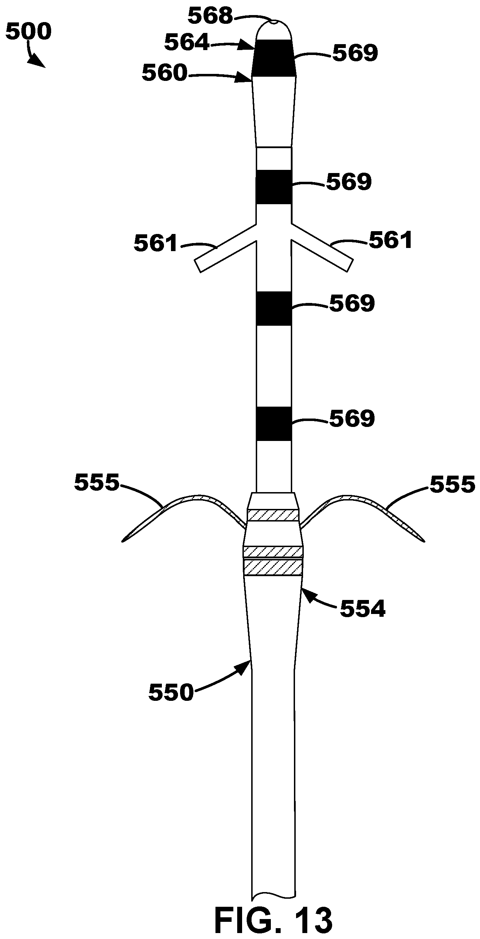

The inner lead body 560 and the outer lead body 550 are depicted in FIG. 12 with the penetration element 520 and the delivery catheter 510 completely removed therefrom. The inner lead body 560 moved distally away from the outer lead body 550, e.g., after the outer lead body 550 is fixated to a target location, is depicted in FIG. 13. As shown, the inner lead body 560 may also include, or define, fixation elements 561 configured to fixate, or fix, the inner lead body 560 within the desired location. In this embodiment, the fixation elements 561 are configured to provide "one-way" fixation. Thus, the fixation elements 561 restrict the inner lead body 560 from moving proximally towards (e.g., back towards) the outer lead body 550 after the inner lead body 560 has been moved away (e.g., distally) from the outer lead body 550. Further, as the inner lead body 560 becomes more exposed, more of the electrodes 569 along the inner lead body 560 are shown.

In brief, the lead and delivery system 500 of FIGS. 10-13 may provide for mechanical injection into the intraseptal space. It may be described that the lead is loaded into a delivery system, a perforating element is extended out of the distal of the lead and is shrouded by the catheter. After lead has been positioned in desired implant location, the lead may be extended out of catheter. As the lead extends out of catheter, the fixation elements (e.g., nitinol tines) may engage the endocardium. Further, as the fixation elements actuate, they extend the lead out of the delivery system. Still further, as the lead tip/needle is actuated forward, the lead is injected into the septum and simultaneously fixated. Then, the needle may be retracted, and the inner assembly of the lead may then be tunneled to desired location. In at least one example, to guide the lead during tunneling, the lead/catheter can be shifted, and shaped/steerable stylets can be used. Further, the inner and outer lead assemblies may then be secured.

An illustrative quadripolar lead 590 that may be used with the systems and methods described herein is depicted FIG. 14. As shown, the lead 590 includes four dipole electrodes 592 spaced along the length of the lead 590. The electrodes 592 may be spaced apart between about 1 millimeters (mm) and about 5 mm. Additionally, the lead 590 includes a plurality of small fixation elements 594 located between each of the electrodes 592. In one embodiment, the fixation elements 594 may be flexible, polymer tines that can fold down in a delivery catheter and can deploy into to muscle fiber when deployed. Also, when enough force is applied, the fixation elements 594 will flex and allow removal chronically (e.g., pulled out of an implant location, moved proximally away from an implant location).

In another embodiment, the lead 590 may include a helix or helical fixation element as opposed to the fixation elements 594 shown in FIG. 14. A helix may allow the lead 590 to be fixated, and then the inner lead body to be advanced after outer lead body is fixated.

In another embodiment, similar to the fixation elements 561 of the inner lead body 560, the fixation elements 594 may be configured to be predominantly "one-way." In other words, the fixation elements 594 may restrict proximal movement of the lead 590 more than distal movement of the lead 590 to, e.g., allow for implantation but restrict dislodgment in the proximal direction.

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, all numbers expressing feature sizes, amounts, and physical properties used in the specification and claims may be understood as being modified either by the term "exactly" or "about." Accordingly, unless indicated to the contrary, the numerical parameters set forth in the foregoing specification and attached claims are approximations that can vary depending upon the desired properties sought to be obtained by those skilled in the art utilizing the teachings disclosed herein or, for example, within typical ranges of experimental error.

Terms related to orientation, such as "proximal," "distal," or "end," are used to describe relative positions of components and are not meant to limit the absolute orientation of the embodiments contemplated.

The terms "coupled" or "connected" refer to elements being attached to each other either directly (in direct contact with each other) or indirectly (having one or more elements between and attaching the two elements). Either term may be modified by "operatively" and "operably," which may be used interchangeably, to describe that the coupling or connection is configured to allow the components to interact to carry out functionality.

The singular forms "a," "an," and "the" encompass embodiments having plural referents unless its context clearly dictates otherwise.

The term "or" is generally employed in its inclusive sense, for example, to mean "and/or" unless the context clearly dictates otherwise. The term "and/or" means one or all of the listed elements or a combination of at least two of the listed elements

The phrases "at least one of," "comprises at least one of," and "one or more of" followed by a list refers to any one of the items in the list and any combination of two or more items in the list.

Thus, various embodiments of implantable medical devices for multi-chamber pacing are disclosed. It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. An implantable medical device comprising:
a plurality of electrodes comprising:
a right atrial electrode (90) positionable within the right atrium to deliver cardiac therapy to or sense electrical activity of the right atrium of the patient's heart; and
at least one left ventricular electrode (92) positionable proximate the left ventricle of the patient's heart to deliver cardiac therapy to or sense electrical activity of the left ventricle of the patient's heart;
a housing (81) extending from a proximal end region (82) to a distal end region (84), wherein the right atrial electrode is leadlessly coupled to the proximal end region;
a leadlet (85) extending from a proximal region (86) to a distal region (87), wherein the proximal region is coupled to the distal end region of the housing and the at least one ventricular electrode is coupled to the distal region of the leadlet, wherein the leadlet is configured to extend through the coronary sinus ostium and into the coronary sinus or a coronary vein of the patient's heart to position the at least one left ventricular electrode proximate the left ventricle of the patient's heart;
a therapy delivery circuit (484) within the housing and operably coupled to the plurality of electrodes to deliver cardiac therapy to the patient's heart;
a sensing circuit (486) within the housing and operably coupled to the plurality of electrodes to sense electrical activity of the patient's heart; and
a controller (480) within the housing and comprising processing circuitry operably coupled to the therapy delivery circuit and the sensing circuit, the controller configured to:
monitor electrical activity using the processing circuitry and one or more of the plurality of electrodes; and
delivering pacing therapy using the processing circuitry and one or more of the plurality of electrodes.

2. The device of claim 1, wherein the at least one left ventricular electrode is implantable into the left ventricular myocardium from the coronary sinus proximal to the posterior vein of the patient's heart.

3. The device of claim 1, wherein the at least one left ventricular electrode is implantable into the anterior interventricular vein of the patient's heart.

4. The device of claim 1, wherein the at least one left ventricular electrode is implantable into the lateral vein of the patient's heart.

5. The device as in any one of claims 1-4, wherein delivering pacing therapy comprises utilizing field steering to avoid left atrial capture by the at least one left ventricular electrode.

## Patentansprüche

1. Implantierbare medizinische Vorrichtung, umfassend:
eine Vielzahl von Elektroden, umfassend:
eine rechtsatriale Elektrode (90), die innerhalb des rechten Atriums positionierbar ist, um die Herztherapie an das rechte Atrium des Herzens des Patienten abzugeben oder um die elektrische Aktivität des rechten Atriums des Herzens des Patienten zu erfassen; und
mindestens eine linksventrikuläre Elektrode (92), die in der Nähe des linken Ventrikels des Herzens des Patienten positionierbar ist, um eine Herztherapie abzugeben oder um die elektrische Aktivität des linken Ventrikels des Herzens des Patienten zu erfassen;
ein Gehäuse (81), das sich von einem proximalen Endbereich (82) zu einem distalen Endbereich (84) erstreckt, wobei die rechtsatriale Elektrode mit dem proximalen Endbereich leitungslos gekoppelt ist;
ein Leadlet (85), das sich von einem proximalen Bereich (86) zu einem distalen Bereich (87) erstreckt, wobei der proximale Bereich mit dem distalen Endbereich des Gehäuses gekoppelt ist und die mindestens eine ventrikuläre Elektrode mit dem distalen Bereich des Leadlet gekoppelt ist, wobei das Leadlet konfiguriert ist, um sich durch das Ostium des Koronarsinus und in den Koronarsinus oder eine Koronarvene des Herzens des Patienten zu erstrecken, um die mindestens eine linksventrikuläre Elektrode in der Nähe des linken Ventrikels des Herzens des Patienten zu positionieren;
eine Therapieabgabeschaltung (484) innerhalb des Gehäuses, und die mit der Vielzahl von Elektroden betriebsfähig gekoppelt ist, um die Herztherapie an das Herz des Patienten abzugeben;
eine Erfassungsschaltung (486) innerhalb des Gehäuses, und die mit der Vielzahl von Elektroden betriebsfähig gekoppelt ist, um die elektrische Aktivität des Herzens des Patienten zu erfassen; und
eine Steuerung (480) innerhalb des Gehäuses, und umfassend eine Verarbeitungsschaltlogik, die mit der Therapieabgabeschaltung und der Erfassungsschaltung betriebsfähig gekoppelt ist, und wobei die Erfassungsschaltung und die Steuerung konfiguriert sind zum:
Überwachen der elektrischen Aktivität unter Verwendung der Verarbeitungsschaltlogik und einer oder mehrerer der Vielzahl von Elektroden. und
Abgeben einer Schrittmachertherapie unter Verwendung der Verarbeitungsschaltlogik und einer oder mehrerer der Vielzahl von Elektroden.

2. Vorrichtung nach Anspruch 1, wobei die mindestens eine linksventrikuläre Elektrode in das linksventrikuläre Myokard von dem Koronarsinus proximal zu der hinteren Vene des Herzens des Patienten implantierbar ist.

3. Vorrichtung nach Anspruch 1, wobei die mindestens eine linksventrikuläre Elektrode für den linken Ventrikel in die vordere interventrikuläre Vene des Herzens des Patienten implantierbar ist.

4. Vorrichtung nach Anspruch 1, wobei die mindestens eine linksventrikuläre Elektrode in die Seitenvene des Herzens des Patienten implantierbar ist.

5. Vorrichtung nach einem der Ansprüche 1 bis 4, wobei das Abgeben einer Schrittmachertherapie das Verwenden einer Feldsteuerung umfasst, um einen linksatrialen Capture durch die mindestens eine linksventrikuläre Elektrode zu vermeiden.

## Revendications

1. Dispositif médical implantable comprenant :
une pluralité d'électrodes comprenant :
une électrode auriculaire droite (90) pouvant être positionnée à l'intérieur de l'oreillette droite pour administrer une thérapie cardiaque ou détecter l'activité électrique de l'oreillette droite du cœur du patient ; et
au moins une électrode ventriculaire gauche (92) pouvant être positionnée à proximité du ventricule gauche du cœur du patient pour administrer une thérapie cardiaque au ventricule gauche du cœur du patient ou détecter l'activité électrique de celui-ci ;
un boîtier (81) s'étendant d'une région d'extrémité proximale (82) à une région d'extrémité distale (84), dans lequel l'électrode auriculaire droite est couplée sans fil à la région d'extrémité proximale ;
une sonde (85) s'étendant d'une région proximale (86) à une région distale (87), dans lequel la région proximale est accouplée à la région d'extrémité distale du boîtier et l'au moins une électrode ventriculaire est couplée à la région distale de la sonde, dans lequel la sonde est conçue pour s'étendre à travers l'ostium du sinus coronaire et dans le sinus coronaire ou une veine coronaire du cœur du patient afin de positionner l'au moins une électrode ventriculaire gauche à proximité du ventricule gauche du cœur du patient ;
un circuit d'administration de thérapie (484) à l'intérieur du boîtier et couplé de manière opérationnelle à la pluralité d'électrodes pour administrer une thérapie cardiaque au cœur du patient ;
un circuit de détection (486) à l'intérieur du boîtier et couplé de manière opérationnelle à la pluralité d'électrodes pour détecter l'activité électrique du cœur du patient ; et
un dispositif de commande (480) à l'intérieur du boîtier et comprenant un circuit de traitement couplé de manière opérationnelle au circuit d'administration de thérapie et au circuit de détection, le dispositif de commande étant configuré pour :
surveiller l'activité électrique à l'aide du circuit de traitement et d'une ou plusieurs parmi la pluralité d'électrodes ; et
administrer une thérapie de stimulation à l'aide du circuit de traitement et d'une ou plusieurs parmi la pluralité d'électrodes.

2. Dispositif selon la revendication 1, dans lequel l'au moins une électrode ventriculaire gauche est implantable dans le myocarde ventriculaire gauche à partir du sinus coronaire proximal à la veine postérieure du cœur du patient.

3. Dispositif selon la revendication 1, dans lequel l'au moins une électrode ventriculaire gauche est implantable dans la veine interventriculaire antérieure du cœur du patient.

4. Dispositif selon la revendication 1, dans lequel l'au moins une électrode ventriculaire gauche est implantable dans la veine latérale du cœur du patient.

5. Dispositif selon l'une quelconque des revendications 1-4, dans lequel l'administration d'une thérapie de stimulation comprend l'utilisation du guidage du champ pour éviter la capture de l'oreillette gauche par l'au moins une électrode ventriculaire gauche.
